# EUROPEAN PATENT APPLICATION

(11) **EP 4 678 744 A1**
(43) Date of publication of application: **14.01.2026**
(21) Application number: 24767422.9
(22) Date of filing: 06.03.2024
(51) Int. Cl.: C12N 9/78, C12N 9/22, C12N 9/24, C12N 15/62, C12N 15/82, A01H 5/10, C12N 15/10

(54) **ADENINE BASE EDITING SYSTEM**

(30) Priority: 06.03.2023 KR 20230029545
(71) Applicant: Greengene, Inc., Seoul 08501 (KR)
(72) Inventor: KIM, Jin Soo, Seoul 08501 (KR); MOK, Young Geun, Seoul 08501 (KR); HONG, Sung Hyun, Seoul 08501 (KR); CHOI, Seung Hee, Seoul 08501 (KR)
(74) Representative: Berggren Oy
(86) International application number: PCT/KR2024/002908
(87) International publication number: WO 2024/186134

(57) **Abstract**

The present invention relates to a base editing composition with the activity to correct adenine (A) bases to guanine (G) bases in DNA, and a base editing method using same. The present invention is useful for correcting bases in nuclear DNA or organellar DNA and particularly for correcting bases in DNA of organelles such as chloroplasts or mitochondria. According to one aspect of the present invention, the base editing composition comprises a DNA-binding protein, cytosine deaminase, adenine deaminase, and uracil DNA glycosylase (UDG). This base editing composition selectively corrects only adenine bases without substantially altering cytosine (C) bases. The present invention also provides a system for correcting adenine bases to guanine bases in plant cell DNA using a single fusion protein comprising a DNA-binding protein, cytosine deaminase, and adenine deaminase.

## Description

### [Technical Field]

The present invention relates to a base editing composition capable of converting an adenine (A) base of DNA to a guanine (G) base, and a base editing method using the same. The present invention is useful for editing bases in nuclear DNA or organellar DNA, and is particularly useful for editing bases in organellar DNA such as chloroplasts or mitochondria. According to one aspect of the present invention, a base editing composition comprises a DNA binding protein, cytosine deaminase, adenine deaminase, and uracil DNA glycosylase (UDG), wherein the base editing composition is capable of selectively converting adenine bases without substantially editing cytosine (C) bases. The present invention also provides a system for converting adenine bases in plant cell DNA into guanine bases using a single fusion protein comprising a DNA binding protein, a cytosine deaminase, and an adenine deaminase.

### [Background Art]

Fusion proteins that link DNA binding proteins and deaminase enzymes enable the induction of DNA mutations, such as single nucleotide conversions in a targeted manner to replace nucleotides or correct bases in the genome without generating DNA double-strand breaks (DSBs), to correct point mutations that cause genetic disorders, or to introduce desired single nucleotide mutations in prokaryotes and eukaryotic cells such as humans.

Programmable genome editing tools, such as zinc-finger nuclease (ZFN), transcription activator-like effector nuclease (TALEN), clustered regularly interspaced short palindromic repeat (CRISPR) system, and base editors composed of CRISPR-associated protein 9 (Cas9) variants and base deaminase proteins, have the potential to be used for plant genetics research and crop trait improvement through base sequence changes. However, these conventional genome editing tools are not suitable for correcting DNA bases in plant organelles, including mitochondria and chloroplasts, particularly because they cannot deliver the guide RNAs required to operate the most widely used CRISPR systems to these organelles. Plant organelles encode many essential genes required for photosynthesis and cellular respiration. Methods or tools for correcting the genes of these organelles are essential for studying the function of these genes and improving crop productivity and traits.

The bacterial toxin DddAtox is the enzymatic component of a bacterial toxin derived from Burkholderia cenocepacia that can deaminate cytosines in double-stranded DNA. Because DddAtox is toxic to cells, it is divided into two inactive splits to avoid toxicity in the host cell. Each split (or half) can be linked to a DNA-binding protein designed to bind to DNA, and used as a cytosine base editor (DdCBE, DddA-derived cytosine base editor) that functions as a pair.

In principle, the enzymatic reaction of this deamination is activated when the two inactive halves are each present close to the target DNA by a DNA-binding protein, and the base conversion from cytosine to thymine (C-to-T) occurs between the two DNA-binding protein binding sites. When a TALE (transcription activator-like effector) protein is used as a DNA binding protein, the base conversion from cytosine to thymine is induced in the region of 14 to 18 bases between two TALE binding sites, and therefore the efficiency of base correction may vary depending on the DNA binding specificity of the TALE protein.

Meanwhile, by linking the DddAtox cytosine deaminase to the DNA binding protein and the adenine deaminase that can cause the correction of adenine to guanine (A-to-G), the adenine base can be corrected. TALE (transcriptional activator-like effector) protein as a DNA binding protein, and a base editor (TALED, TALE-linked deaminase) that links cytosine deaminase and adenine deaminase to it, can introduce a wide spectrum of mutations, unlike cytosine base editors that only cause C-to-T correction.

### [Disclosure]

### [Technical Problem]

Since conventional TALED has cytosine deaminase in addition to adenine deaminase, the result is that not only adenine bases but also cytosine bases present on the target DNA are corrected. This was confirmed experimentally to be particularly prominent in the chloroplasts of plants. Since there is a need to selectively edit only the adenine base while maintaining the cytosine base sequence as it is in the wild type, the inventors of the present invention sought to develop a method that can selectively correct only the adenine base to the guanine base without causing correction of unwanted cytosine bases.

Meanwhile, since the DddAtox cytosine deaminase is used in the form of two inactivated splits to avoid toxicity, two TALE modules are required in which the two splits are each connected so that each of the two splits can be positioned close to the target DNA requiring correction. However, using two TALE modules in this way has disadvantages such as limitations in selecting the target site for DNA correction and the need for a large vector size for expressing the base editor. Accordingly, the inventors of the present invention sought to provide a method for correcting adenine bases in plant cell DNA using monomeric TALED linked to a full-length DddAtox cytosine deaminase with no toxicity. In addition, it is another object of the present invention to provide a method for selectively converting adenine bases without causing correction of unwanted cytosine bases by using monomeric TALED linked to such full-length DddAtox cytosine deaminase.

### [Technical Solution]

The present invention provides a base editing composition capable of converting an adenine base into a guanine base, the base editing composition further comprising uracil DNA glycosylase (UDG) in addition to a DNA binding protein, a cytosine deaminase, and an adenine deaminase. The base editing composition has an activity of converting adenine bases without substantially converting cytosine bases, thereby converting the adenine bases into guanine bases. By using such a base editing composition, a base editing system capable of selectively converting adenine bases can be provided, which can be used to convert not only nuclear DNA but also organellar DNA such as chloroplasts or mitochondria. The base editing composition of the present invention can be used for both plant cells and animal cells, and in particular, can be used to obtain a plant or a seed thereof in which only adenine bases of a desired target DNA are selectively editied into guanine bases by transforming a plant cell with the base editing composition of the present invention.

The present invention also provides a base editing composition for converting an adenine base in a plant cell into a guanine base, comprising a DNA binding protein, a cytosine deaminase, and an adenine deaminase, wherein the cytosine deaminase is included in a full-length form rather than in a split form. Using the base editing composition eliminates the need for a fusion protein comprising two or more DNA binding proteins (preferably TALE proteins) and a deaminase, thereby allowing unrestricted selection of target sites for DNA editing.

### [Description of Drawings]

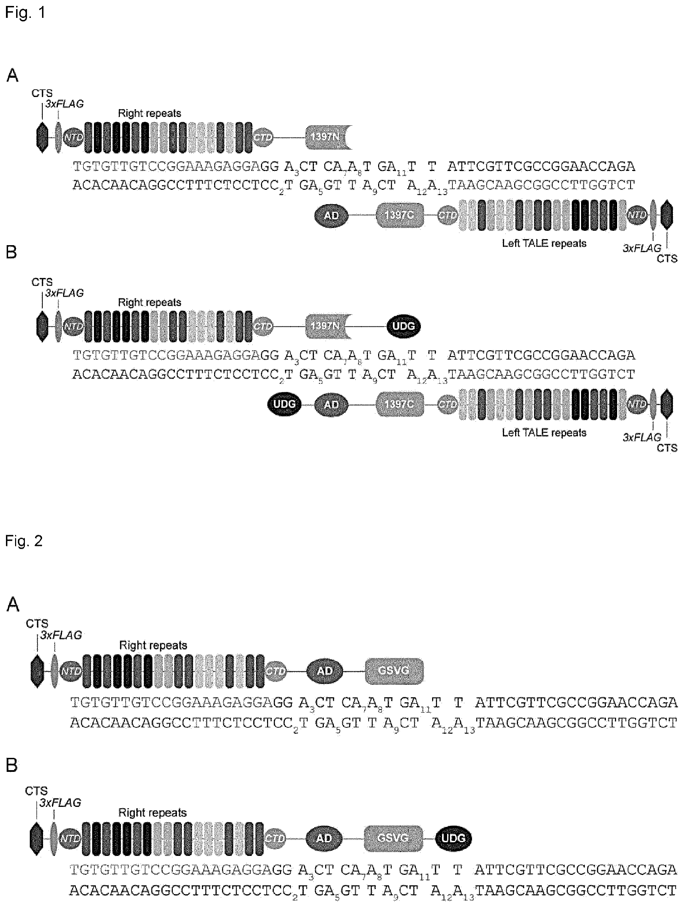

FIG. 1 is a schematic diagram of a base editor targeting the plant chloroplast gene psaA using a DddAtox split, showing the case without UDG (A) and the case with UDG (B). CTS represents a chloroplast transit signal, AD represents adenine deaminase, NTD represents the N-terminal domain, CTC represents the C-terminal domain, and UDG represents uracil DNA glycosylase. "Right TALE repeats" and "Left TALE repeats" represent TALE arrays, respectively, and the base sequences indicated in blue represent the binding sites of the TALE protein (consisting of the N-terminal domain, the TALE array, and the C-terminal domain). Base editing occurs in the DNA sequence region between the binding sites of two TALE proteins, mediated by deaminase enzymes.
FIG. 2 is a schematic diagram of a base editor targeting the plant chloroplast gene psaA using full-length DddAtox GSVG, showing the cases without UDG (A) and with UDG (B). GSVG refers to a variant (SEQ ID NO: 9) of cytosine deaminase having the amino acid sequence of SEQ ID NO: 2, in which S at position 37, G at position 59, A at position 109, and S at position 129 are substituted with G, S, V, and G, respectively.

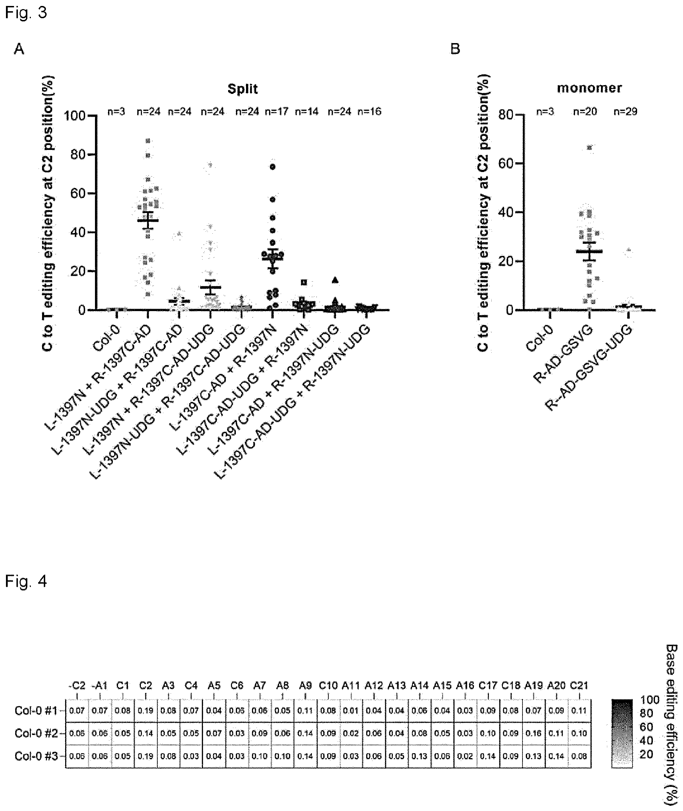

FIG. 3 shows the base editing efficiency at C2, the position in the psaA target DNA where conversion of cytosine occurs, in a first-generation plant transformed with a base editor according to the present invention. C2 refers to cytosine, the second base in the DNA sequence to be converted, located between the first TALE protein (left TALE; SEQ ID NO: 10) and the second TALE protein (right TALE; SEQ ID NO: 11) of the base editing composition used as shown in FIGS. 1 and 2. Col-0 refers to a non-transformed wild-type plant. Drawing A shows the case using a pair of TALEs linked to DddAtox splits, and Drawing B shows the case using monomeric TALEs linked to full-length DddAtox. Col-0 is a non-transformed wild-type individual. "L" represents the left TALE having the amino acid sequence of SEQ ID NO: 10, and "R" represents the right TALE having the amino acid sequence of SEQ ID NO: 11. "AD" represents adenine deaminase having the amino acid sequence of SEQ ID NO: 1. "GSVG" represents full-length DddAtox GSVG having the amino acid sequence of SEQ ID NO: 9.
FIG. 4 shows the base editing rate at the psaA target site of three wild-type plants (Col-0). Since it should theoretically be zero, the displayed value may be interpreted as due to sequencing errors and considered a reference value for determining whether actual base editing has occurred.
FIG. 5 shows the base editing efficiency of the psaA target DNA in 24 first-generation plants transformed with a base editor using a pair of TALEs in which the DddAtox split was used and UDG was not used. L-1397N represents the left TALE linked to the N-terminal DddAtox split, and R-1397C-AD represents the right TALE linked to the C-terminal DddAtox split and adenine deaminase.
FIG. 6 shows the base editing efficiency of the psaA target DNA in 24 first-generation plants transformed with a base editor using a pair of TALEs in which UDG is linked to the left TALE and adenine deaminase is linked to the right TALE, using the DddAtox split.
FIG. 7 shows the base editing efficiency of the psaA target DNA in 24 first-generation plants transformed with a base editor using a pair of TALEs in which adenine deaminase and UDG are linked to the right TALE, using the DddAtox split.
FIG. 8 shows the base editing efficiency of the psaA target DNA in 24 first-generation plants transformed with a base editor using a pair of TALEs in which UDG is linked to the left TALE and adenine deaminase and UDG are linked to the right TALE, using the DddAtox split.
FIG. 9 shows the base editing efficiency of the psaA target DNA in 17 first-generation plants transformed with a base editor using a pair of TALEs in which adenine deaminase is linked to the left TALE, with the DddAtox split used and UDG not used.
FIG. 10 shows the base editing efficiency of the psaA target DNA in 14 first-generation plants transformed with a base editor using a pair of TALEs in which adenine deaminase and UDG are linked to the left TALE, using the DddAtox split.
FIG. 11 shows the base editing efficiency of the psaA target DNA in 24 first-generation plants transformed with a base editor using a pair of TALEs in which adenine deaminase is linked to the left TALE and UDG is linked to the right TALE, using the DddAtox split.
FIG. 12 shows the base editing efficiency of the psaA target DNA in 16 first-generation plants transformed with a base editor using a pair of TALEs in which the left TALE is linked to adenine deaminase and UDG, and the right TALE is linked to UDG, using the DddAtox split.
FIG. 13 shows the base editing efficiency of the psaA target DNA in 20 first-generation plants transformed with a monomeric TALED base editor not containing UDG, using a full-length DddAtox variant.
FIG. 14 shows the base editing efficiency of the psaA target DNA in 29 first-generation plants transformed with a UDG-linked monomeric TALED base editor using a full-length DddAtox variant.

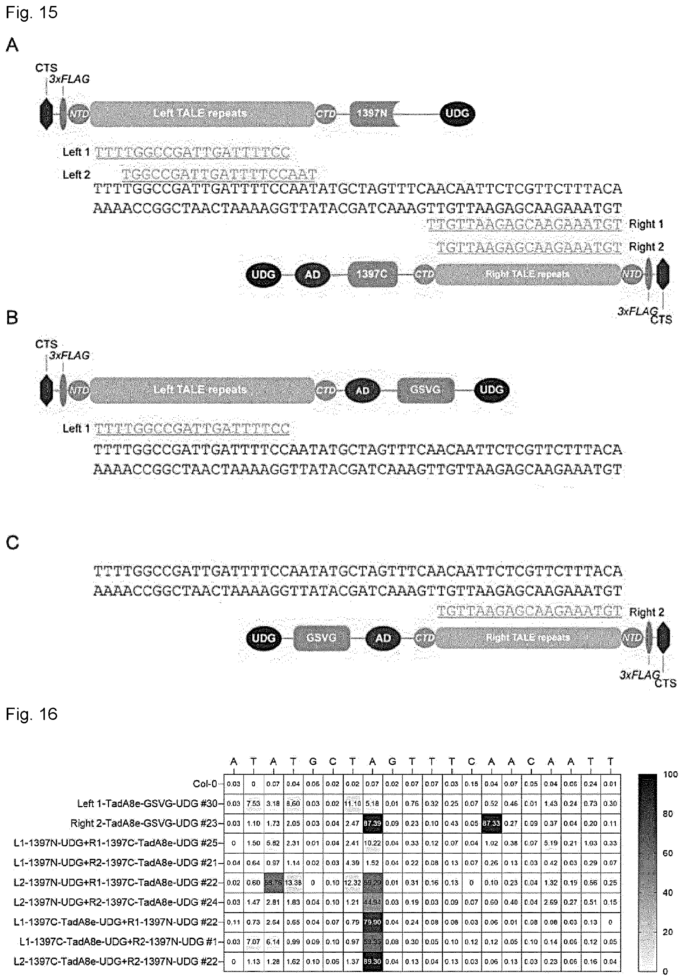

FIG. 15 is a schematic diagram of base editors targeting the plant chloroplast gene psbA using DddAtox splits or full-length DddAtox (SEQ ID NO: 9). CTS represents the chloroplast transit signal, NTD represents the N-terminal domain of the TALE protein, and CTD represents the C-terminal domain of the TALE protein. "Left TALE repeats" and "Right TALE repeats" represent TALE arrays, respectively, and the base sequences underlined in blue represent the binding sites of the TALE protein (consisting of the N-terminal domain, the TALE array, and the C-terminal domain). Base editing occurs in the DNA sequence region between the binding sites of two TALE proteins, mediated by deaminase enzymes. FIG. 15A uses DddAtox splits (1397N and 1397C), while FIGS. 15B and 15C use full-length DddAtox ("GSVG"; SEQ ID NO: 9), using only the Left TALE repeats or only the Right TALE repeats, respectively.
FIG. 16 shows the results of measuring the base editing efficiency for each individual that survived atrazine treatment among the first-generation plants transformed with the base editors shown in FIG. 15. Col-0 is a non-transformed wild-type individual. "Left 1" and "L1" refer to the left TALE protein having the amino acid sequence of SEQ ID NO: 14. "L2" refers to the left TALE sequence of SEQ ID NO: 15. "R1" refers to the right TALE protein of SEQ ID NO: 16. "Right 2" and "R2" refer to the right TALE protein of SEQ ID NO: 17.

### [Modes for Carrying Out the Invention]

Unless defined otherwise, all technical and scientific terms used herein have the same meaning as commonly understood by those of ordinary skill in the art to which the present invention pertains. In general, the terms used in this specification are well known and commonly used in the art.

The terms "correction," "editing," and "editing (Korean transliteration of 'editing')," as used herein, are used interchangeably and refer to a method of altering a nucleic acid sequence by selective mutation of a specific genomic target. Such specific genomic targets include, but are not limited to, genes, promoters, open reading frames, or any nucleic acid sequence.

The terms "base editor," "base editing system," and "base correction system (Korean translation of 'base editing system')," as used herein, are used interchangeably and mean a substance capable of editing a nucleic acid sequence by selective mutation of a genomic target, and include a combination of one or more different base editors. The terms "base editor," "base editing system," or "base correction system," as used herein, may refer to a polypeptide (which may be a fusion protein), a polynucleotide, or a combination thereof, depending on the context, or may refer to a composition comprising one or more polypeptides (which may be fusion proteins), polynucleotides, or a combination thereof. Therefore, the term "base correction system" or "base editing composition," as used herein, may include one base editor or a combination of two or more different base editors, wherein the different base editors may be used simultaneously or separately.

The term "fusion protein," as used herein, refers to a polypeptide formed by joining two or more different polypeptides via peptide bonds. The individual proteins constituting the fusion protein according to the present invention are typically cloned into a single polynucleotide and expressed as a single polypeptide (fusion protein), but one or more of the individual proteins is cloned into separate polynucleotide(s) and expressed as two or more separate polypeptides, and such cases are also within the scope of the present invention. The term "linker" refers to any molecule that connects two different molecules, and any linker known to be usable in providing a fusion protein or protein conjugate in the field of biotechnology may be used, for example, a peptide linker comprising 1 to 100 amino acid residues.

The method for designing and constructing the fusion protein or the polynucleotide encoding it is any method known in the art, the polynucleotide is inserted into a vector, and the vector is introduced into a cell. The individual proteins constituting the fusion protein according to the present invention are typically cloned into a single polynucleotide and expressed as a single polypeptide (fusion protein), but one or more of the individual proteins is cloned into separate polynucleotide(s) and expressed as two or more separate polypeptides, and such cases are also within the scope of the present invention.

The terms "target," "target region," or "target site" as used herein mean a pre-identified nucleic acid sequence of any composition and/or length. Such target sites include, but are not limited to, genes, promoters, open reading frames, or any nucleic acid sequence.

The present invention provides a base editing composition capable of converting an adenine base of DNA to guanine, wherein the base editing composition comprises one or more fusion proteins, each fusion protein comprising a DNA binding protein and a cytosine deaminase, wherein at least one of the fusion proteins comprises an adenine deaminase, and wherein at least one of the fusion proteins comprises UDG. Here, the cytosine deaminase is present in the form of full-length or as two split forms.

The base editing composition according to the present invention is characterized by including both cytosine deaminase and adenine deaminase. In particular, a base editor that connects both cytosine deaminase and adenine deaminase using TALE protein as a DNA binding protein is called TALED. Unlike previous DdCBE base editors that performed conversion only of cytosine bases among specific motifs in organellar DNA, TALED enables base conversion from A to G. In the specification of the present application, the term "TALED" means a base editor in which both a cytosine deaminase and an adenine deaminase are linked to a TALE protein, wherein the TALE protein may be used in the form of a monomer (for example, when the cytosine deaminase is used in a full-length form) or in the form of a paired dimer (for example, when the cytosine deaminase is used in a split form), and the adenine deaminase may be linked to both of the TALE proteins or to only one TALE protein.

The base editing composition according to the present invention may be used to edit not only nuclear DNA but also DNA of cellular organelles such as chloroplasts or mitochondria. The base editing composition of the present invention can be used for both plant cells and animal cells, and in particular, can be used to obtain a plant or a seed thereof in which only adenine bases of a desired target DNA are selectively editied into guanine bases by transforming a plant cell with the base editing composition of the present invention.

The cytosine deaminase used in the present invention may be included in the form of a first split and a second split, and the first split and the second split are each in a form bound to a DNA binding protein.

The cytosine deaminase that may be used in the base editor according to the present invention refers to an amino group deaminase capable of converting a cytosine base to a uridine, and may be derived from or mutated (e.g., engineered or evolved) from any organism (e.g., eukaryote or prokaryote) including, but not limited to, algae, bacteria, fungi, plants, invertebrates, and mammals. For example, it may be a cytosine deaminase derived from or mutated from APOBEC (apolipoprotein B editing complex), AID (activation-induced deaminase), TadA (tRNA-specific adenosine deaminase), a bacterial adenine deaminase, or an ortholog thereof, or DddA, a bacterial cytosine deaminase, or a cytosine deaminase derived from or mutated from an ortholog thereof, or a split thereof. The cytosine deaminase mutated from the above-mentioned TadA may be, for example, one in which one or more of the amino acid residues 6, 26, 27, 28, 46, 48, 49, 61, 74, 76, 77, 82, 96, 107, 108, 112, 114, 115, 119, 122, 127, 142, 143, 151, 154 and 158 in the amino acid sequence of SEQ ID NO: 1 are mutated to different amino acid. For example, it may be a polypeptide in which the 27th amino acid in the amino acid sequence of SEQ ID NO: 1 is mutated to lysine, the 28th amino acid is mutated to alanine, the 61st amino acid is mutated to isoleucine, and the 96th amino acid is mutated to asparagine. Regarding the composition of the cytosine deaminase that may be used in the present invention, reference may be made to international patent application publications WO 2022/060185, WO 2023/086953, etc., which are incorporated by reference in their entirety into this application, as well as to contents already known prior to the present application.

When a cytosine deaminase is included in the base editor according to the present invention, the cytosine deaminase may be included in the form of a first split and a second split, or may also be included in a full-length form. When in the form of a first split and a second split, the first split and the second split may be linked to respective DNA binding proteins.

In this specification, when two proteins are said to be "linked," they may be directly linked or indirectly linked via a linker or other protein(s).

The cytosine deaminase used in the present invention may be DddAtox, which is a portion of a bacterial toxin derived from Burkholderia cenocepacia that exhibits an enzymatic function and is capable of deaminating cytosine in double-stranded DNA. DddAtox may comprise the amino acid sequence of SEQ ID NO: 2.

SEQ ID NO: 2: wild-type DddAtox

GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG

AIPVKRGATGETKVFTGNNSNSPKSPTKGGC

Because DddAtox is toxic to cells, it may be used in the form of two inactive splits, namely the first split and the second split, to avoid toxicity in host cells. When the cytosine deaminase used in the present invention is used in the form of a first split and a second split, each of the first split and the second split does not have deamination activity.

The first split of the DddAtox cytosine deaminase may include a sequence from the N-terminus to G33, G44, A54, N68, G82, N98, or G108 of the amino acid sequence of SEQ ID NO: 2, and the second split may include a sequence from G34, P45, G55, N69, T83, A99, or A109 of the amino acid sequence of SEQ ID NO: 2 to the C-terminus.

Preferably, the first split of the DddAtox cytosine deaminase may include a sequence from the N-terminus to G44 (SEQ ID NO: 3 below) of the amino acid sequence of SEQ ID NO: 2, and the second split may include a sequence from P45 to the C-terminus (SEQ ID NO: 4 below).

SEQ ID NO: 3: wild-type DddAtox G1333-N

GSYALGPYQISAPQLPAYNGQTVGTFYYVNDAGGLESKVFSSGG

SEQ ID NO: 4: wild-type DddAtox G1333-C

PTPYPNYANAGHVEGQSALFMRDNGISEGLVFHNNPEGTCGFCVNMTETLLP
ENAKMTVVPPEGAIPVKRGATGETKVFTGNNSNSPKSPTKGGC

Preferably, the first split of the DddAtox cytosine deaminase may include a sequence from the N-terminus to G108 of the amino acid sequence of SEQ ID NO: 2 (SEQ ID NO: 5 below), and the second split may include a sequence from A109 to the C-terminus (SEQ ID NO: 6 below).
SEQ ID NO: 5: wild-type DddAtox G1397-N 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
SEQ ID NO: 6: wild-type DddAtox G1397-C AIPVKRGATGETKVFTGNNSNSPKSPTKGGC

When the first and second splits of DddAtox are used as cytosine deaminase, one or more amino acids located at the surface where the first and second splits bind to each other may be substituted with different amino acids. For example, the first split and the second split of DddAtox may each comprise the amino acid sequences of SEQ ID NO: 3 (G1333-N) and SEQ ID NO: 4 (G1333-C), in which case one or more amino acids selected from the group consisting of positions 3, 5, 10, 11, 13, 14, 15, 16, 17, 18, 19, 28, 30 and 31 of SEQ ID NO: 3 or one or more amino acids selected from the group consisting of positions 13, 16, 17, 20, 21, 28, 29, 30, 31, 32, 33, 56, 57, 58 and 60 of SEQ ID NO: 4 may be substituted with different amino acids, but is not limited thereto. As another example, the first split of DddAtox may comprise the amino acid sequence of SEQ ID NO: 5 (G1397-N) and SEQ ID NO: 6 (G1397-C), wherein one or more amino acids selected from the group consisting of positions 87, 88, 91, 92, 95, 100, 101, 102 and 103 of SEQ ID NO: 5 or one or more amino acids selected from the group consisting of positions 13, 14, 15 and 16 of SEQ ID NO: 6 may be substituted with different amino acids, but is not limited thereto. The above "different amino acids" means amino acids selected from among alanine, isoleucine, leucine, methionine, phenylalanine, proline, tryptophan, valine, aspartic acid, cysteine, glutamine, glycine, serine, threonine, tyrosine, aspartic acid, glutamic acid, arginine, histidine, lysine, and all known variants of the above amino acids, excluding the amino acid that the wild-type protein originally has at the mutation position. This variant enables highly efficient and precise C-to-T correction without causing undesirable off-target C-to-T correction, as the two DddAtox splits, each linked to a DNA-binding protein, do not function properly if they fail to bind DNA.

In the present specification, the term "G1333-N", "G1333N" or "1333N" may refer to a first split of wild-type DddAtox having an amino acid sequence of SEQ ID NO: 3 or an amino acid variant thereof, and the term "G1333-C", "G1333C" or "1333C" may refer to a second split of wild-type DddAtox having an amino acid sequence of SEQ ID NO: 4 or an amino acid variant thereof.

In the present specification, the term "G1397-N", "G1397N" or "1397N" may refer to a first split of wild-type DddAtox having an amino acid sequence of SEQ ID NO: 5 or an amino acid variant thereof, and the term "G1397-C", "G1397C" or "1397C" may refer to a second split of wild-type DddAtox having an amino acid sequence of SEQ ID NO: 6 or an amino acid variant thereof.

The cytosine deaminase used in the present invention may be used in a full-length form, and the full-length cytosine deaminase used in this case (e.g., DddAtox) is modified so as to have no toxicity or only low toxicity. The C-terminus of DddAtox is specifically enriched with positively charged amino acids. Because DNA is negatively charged, it binds to amino acids that have positive charge properties in proteins. By substituting these positively charged amino acids, the binding strength of DddAtox to DNA may be weakened, thereby reducing or eliminating intracellular toxicity. That is, by substituting the positively charged amino acids so as to make the protein non-toxic, cloning using E. coli is possible, thereby obtaining the full-length DddAtox. Such non-toxic full-length cytosine deaminase may be provided by substituting one or more, two or more, three or more, or five or more amino acids of the wild-type amino acid sequence of SEQ ID NO: 2 with different amino acids. The term "different amino acids" refer to amino acids selected from alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, lysine, and all known variants thereof, excluding the amino acid that the wild-type protein naturally has at the mutation position. For example, the different amino acids may be alanine.

The non-toxic full-length DddAtox may include an amino acid sequence selected from the group consisting of the following amino acid sequences.
A1341D KRKKA variant 
GSYALGPYQISAPQLPAYNGQTVGTFYYVNDAGGLESKVFSSGGPTPYPNYD
NAGHVEGQSALFMRDNGISEGLVFHNNPEGTCGFCVNMTETLLPENAKMTVVPPEG
AIPVKRGATGETKVFTGNNSNSPKSPTAGGC
AAAAA variant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETAVFTGNSNSPASPTAGGC
AAAAK mutant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETAVFTGNSNSPASPTKGGC
AAKAA mutant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETKVFTGNSNSPASPTAGGC
AAKAK mutant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVAAGATGETKVFTGNSNSPASPTKGGC
KAAAA mutant 
GSYALGPYQISAPQLPAYNGQTVGTFYYVNDAGGLESKVFSSGGPTPYPNYA
NAGHVEGQSALFMRDNGISEGLVFHNNPEGTCGFCVNMTETLLPENAKMTVVPPEG
AIPVKAGATGETAVFTGNSNSPASPTAGGC
E1347A variant

GSYALGPYQISAPQLPAYNGQTVGTFYYVNDAGGLESKVFSSGGPTPYPNYA
NAGHVAGQSALFMRDNGISEGLVFHNNPEGTCGFCVNMTETLLPENAKMTVVPPEG
AIPVKRGATGETKVFTGNNSNSPKSPTKGGC

Preferably, the full-length cytosine deaminase variant used in the present invention may have one or more amino acid substitutions selected from the group consisting of a substitution of S at position 37 with G, a substitution of G at position 59 with S, a substitution of A at position 109 with V, and a substitution of S at position 129 with G in the amino acid sequence of SEQ ID NO: 2.

More preferably, the full-length cytosine deaminase variant used in the present invention may have all of the following substitutions in the amino acid sequence of SEQ ID NO: 2: substitution of S at position 37 with G, substitution of G at position 59 with S, substitution of A at position 109 with V, and substitution of S at position 129 with G, in which case the sequence is as follow.

GSVG variant

GSYALGPYQISAPQLPAYNGQTVGTFYYVNDAGGLEGKVFSSGGTPPYPNYA
NAGHVESQSALFMRDNGISEGLVFHNNPEGTCGFCVNMTETLLPENAKMTVVPPEG
VIPVKRGATGETKVFTGNSNGPKSPTKGGC

As another example, a full-length cytosine deaminase variant used in the present invention may comprise the following sequence:
SSVG variant 
GSYALGPYQISAPQLPAYNGQTVGTFYYVNDAGGLESKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC
GSAG mutant 
GSYALGPYQISAPQLPAYNGQTVGTFYYVNDAGGLEGKVFSSGGTPPYPNYA
NAGHVESQSALFMRDNGISEGLVFHNNPEGTCGFCVNMTETLLPENAKMTVVPPEG
AIPVKRGATGETKVFTGNSNGPKSPTKGGC
GSVS mutant

GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC

The adenine deaminase used in the base editor according to the present invention refers to an amino group deaminase capable of converting an adenine base into hypoxanthine (inosine as a nucleoside), and may be derived from or mutated (e.g., engineered or evolved) from any organism (e.g., eukaryote or prokaryote), including but not limited to algae, bacteria, fungi, plants, invertebrates, and mammals, for example, E. coli, S. aureus, S. typhi, S. putrefaciens, H. influenzae, or C. crescentus. Such adenine deaminase may be, for example, APOBEC, AID, TadA, or a variant thereof. The above-mentioned TadA may be, for example, TadA8e (SEQ ID NO: 1) or a truncated form or variant thereof (e.g., a variant engineered or evolved to be applicable to deoxynucleotides). The above-mentioned variant of TadA8e may be, for example, one in which one or more of amino acid residues 23, 28, 30, 36, 46, 48, 49, 51, 76, 82, 82, 84, 106, 108, 110, 111, 146, 147, 152, 154, 155, 156, and 157 of SEQ ID NO: 1 are substituted with different amino acids. Regarding the composition of the adenine deaminase used in the present invention, reference may be made to International Patent Application Publications WO 2022/060185, WO 2023/086953, etc., which are incorporated by reference in their entirety into this application, as well as to contents already known prior to the present application. The adenine deaminase used in the present invention may be provided as a base editor composition comprising the amino acid sequence of SEQ ID NO: 1 or a conservative amino acid substitution thereof.

When cytosine deaminase is used in a split form in the base editor according to the present invention, adenine deaminase may be linked to the N-terminus or C-terminus of the first split of the cytosine deaminase, and/or to the N-terminus or C-terminus of the second split.

Regarding the composition of the adenine deaminase used in the present invention, reference may be made to International Patent Application Publications WO 2022/060185, WO 2023/086953, etc., which are incorporated by reference in their entirety into this application, as well as to contents already known prior to the present application.

The DNA binding protein according to the present invention may be a zinc finger protein, a TALE protein, a CRISPR-associated nuclease, or a combination thereof. With respect to the composition of zinc finger proteins, TALE proteins and CRISPR-associated nucleases, reference may be made to the contents already known prior to the present application, including International Patent Application Publication No. WO2022/060185, which is incorporated by reference herein in its entirety.

The DNA binding protein according to the present invention may be a TALE protein. The TALE protein of the present invention refers to a protein that binds to nucleotides in a sequence-specific manner via one or more TALE-repeat modules. The TALE protein comprises at least one TALE-repeat module, preferably 1 to 30 TALE-repeat modules, but is not limited thereto. As used herein, TALE-repeat modules may be referred to as a "TALE array", and the term "TALE protein" refers to a configuration comprising an N-terminal domain and a C-terminal domain (which may include a half domain) on each side of the TALE array. The term "TALE" as used herein may mean only "TALE array" or "TALE protein" depending on the context.

When a TALE protein is used as the DNA binding protein of the base editor according to the present invention, a single module TALE array or a multi-module TALE array (e.g., a dual module TALE array of a first TALE array and a second TALE array) may be used. For example, a first TALE protein (left TALE) may be bound to a first split of cytosine deaminase (first fusion), and a second TALE protein (right TALE) may be bound to a second split of the cytosine deaminase (second fusion). In this case, the first TALE protein (left TALE) is linked to the first split of cytosine deaminase in the N-C direction, and the second TALE protein (right TALE) is linked to the second split of cytosine deaminase, and the first TALE protein (left TALE) and the second TALE protein (right TALE) are respectively linked in the structure of an N-terminal domain, a TALE array, and a C-terminal domain (which may include a half domain). The first split may be an N-terminal split or a C-terminal split of a full-length cytosine deaminase, and the second split may also be an N-terminal split or a C-terminal split of a full-length cytosine deaminase. Either a single-module TALE array or a multi-module TALE array may be used, even if the cytosine deaminase is full-length. When a single-module TALE array is used, a single TALE domain and cytosine deaminase are linked in the N-C direction. When a dual-module TALE is used, the first TALE protein (left or right TALE) is linked to the full-length cytosine deaminase in the N-C direction, and the second TALE protein (left or right TALE) may be included separately.

When cytosine deaminase is used in the form of a first split and a second split, and the DNA binding protein is a TALE protein, the base editor of the present invention may be in the form of a composition comprising a first fusion in which a first TALE is linked to the first split of the cytosine deaminase, and a second fusion in which a second TALE is linked to the second split of the cytosine deaminase. The first and second fusions have the structures N'-TALE-first split (cytosine deaminase)-C' and N'-TALE-second split (cytosine deaminase)-C', respectively. The adenine deaminase may be linked to the first fusion or the second fusion, or both, and specifically to the N-terminus or the C-terminus of the first split of the cytosine deaminase in the first fusion, and/or to the N-terminus or the C-terminus of the second split of the cytosine deaminase in the second fusion.

When cytosine deaminase is used in a full-length form and the DNA binding protein has the form of a single TALE module as a TALE protein, the single TALE module and cytosine deaminase are linked in the N-to-C direction, and at this time, the adenine deaminase is connected to the C-terminal side of the single TALE module and may be linked to the N-terminus or C-terminus of the cytosine deaminase. In the specification of the present application, the expression "monomeric TALED" refers to a form in which a single TALE module is used as a DNA binding protein, and cytosine deaminase and adenine deaminase are linked.

When the cytosine deaminase is used in a full-length form and the DNA binding protein is a TALE protein in the form of a dual TALE module, the base editor of the present invention may have the form of a composition comprising a first fusion in which a first TALE module and cytosine deaminase are linked in the N-to-C direction, and a second fusion comprising an adenine deaminase and a second TALE. The first and second fusions have the structures of N'-TALE-cytosine deaminase-C' and N'-TALE-adenine deaminase-C', respectively, and the adenine deaminase may be bound to the N-terminus or C-terminus of TALE.

According to one embodiment of the present invention, the base editing composition of the present invention is characterized by comprising uracil DNA glycosylase (UDG). Specifically, at least one of the fusion proteins included in the base editing composition according to the present invention comprises UDG. UDG is known to be capable of recognizing damaged DNA in its natural state and selectively removing only uracil bases within the DNA. Since the A-to-G base editor according to the present invention includes a cytosine deaminase such as DddAtox, the cytosine base is deaminated and converted to uracil. When UDG is expressed together, the converted uracil base is removed and the original DNA sequence is restored, so as to avoid unwanted cytosine base editing. This technique may be particularly useful for selectively converting adenine bases in environments where the natural expression of UDG is relatively low, particularly in plant cell organelles. Any species of UDG may be used, for example, UDG from Arabidopsis, human, mouse, tobacco, or rice, and the UDG may have various lengths, for example, 2, 5, 10, 16, 24, or 32 amino acids. For example, the UDG used in the present invention may include, but is not limited to, the amino acid sequence of SEQ ID NO: 7.

UDG is preferably used in the form of a fusion protein together with the DNA binding protein, cytosine deaminase and adenine deaminase used in the present invention, but may also be delivered to the target DNA in a form (protein or polynucleotide) separate from the above components. When used in the form of a fusion protein, UDG may be linked to the C-terminus of cytosine deaminase (or a split thereof) or the C-terminus of adenine deaminase, but is not necessarily limited thereto.

According to one embodiment of the present invention, a base editor according to the present invention comprising UDG may selectively edit only adenine bases of DNA without substantially editing cytosine bases to thymine bases. For example, a UDG-linked TALED according to the present invention may cause editing of cytosine bases to thymine at a frequency of less than 20%, less than 10%, less than 5%, or less than 1%. The above base editing frequency (or efficiency) may be measured as the base editing frequency (or efficiency) calculated when sequencing target DNA from a cell transformed to express the base editor according to the present invention, and may be expressed as, for example, the percentage value of sequencing reads that reflect the results of the desired base editing among the total sequencing reads for DNA obtained from the base-edited cell or individual.

At least one of the fusion proteins included in the base editing composition according to the present invention may additionally include a nuclear export signal (NES). When NES is attached to a base-editing protein, bases may be edited with higher efficiency. The above NES sequence may be any signal sequence (e.g., VDEMTKKFGTLTIHDTEK) that confers the ability to translocate outside the nucleus, and a natural NES or an artificially synthesized NES may be used. For example, it may be derived from, but is not limited to, mirut virus of mice (MVM).

At least one of the fusion proteins included in the base editing composition according to the present invention may additionally include a mitochondrial targeting sequence (MTS). The MTS that can be used in the present invention may be any signal sequence capable of translocating into mitochondria, and may be a natural MTS present at the N-terminus of various mitochondrial proteins, or an artificially synthesized MTS. When MTS is used in the base editing composition according to the present invention, its location may vary, and may be linked directly or indirectly (e.g., via a linker and/or other protein component) to the N-terminus of a DNA binding protein or the N-terminus of a NES, for example, but is not limited thereto.

At least one of the fusion proteins included in the base editing composition according to the present invention may additionally comprise a chloroplast transit signal (CTS), and such a base editing composition may be useful for editing chloroplast, chromoplast, or leucoplast DNA of a plant cell. The CTS that can be used in the present invention may be any signal sequence capable of translocating into a chloroplast, and may be a natural CTS present at the N-terminus of various chloroplast proteins, or an artificially synthesized CTS may be used. When CTS is used in the base editing composition according to the present invention, its location may vary, and may be linked directly or indirectly (e.g., via a linker and/or other protein components) to the N-terminus of a DNA binding protein or the N-terminus of a NES, but is not limited thereto.

At least one of the fusion proteins included in the base editing composition according to the present invention may comprise a nuclear localization signal (NLS). The NLS that can be used in the present invention may be any signal sequence capable of translocating into the nucleus, and may be a natural NLS present at the N-terminus of various nuclear proteins, or an artificially synthesized NLS may be used. When NLS is used in the base editing composition according to the present invention, its location may vary, and may be linked directly or indirectly (e.g., via a linker and/or other protein components) to the N-terminus of a DNA binding protein, but is not limited thereto.

The base editor according to the present invention is in the form of a fusion protein. Specifically, the fusion protein comprises a DNA binding protein and a deaminase or a split thereof, may further include auxiliary sequences such as UDG, NLS, NES, MTS, or CTS, and may include additional sequences such as tags for biotechnological techniques, and each of these polypeptides may be directly linked or linked via a linker. Such fusion proteins (or polynucleotides encoding fusion proteins) may be designed and produced using any method known in the field of biotechnology.

In the present invention, the base editor may be in the form of a polynucleotide encoding a fusion protein described in the specification of the present application. Such polynucleotides may be inserted into a vector, and the vector may be introduced into a cell.

The present invention also provides plant cells transformed with the vector, plants grown therefrom, progeny or clones of the plants, and seeds obtained from such plants.

The plant cells, plants, and seeds according to the present invention are characterized in that the adenine base of wild-type DNA is corrected to guanine. In particular, plant cells, plants, and seeds transformed with a base editor containing UDG (or a polynucleotide encoding the same) may be characterized in that adenine bases of wild-type DNA are selectively corrected while cytosine bases remain uncorrected.

The present invention also provides a method of converting an adenine base of DNA to a guanine base, comprising expressing a base editing composition described in the specification of the present application or a polynucleotide encoding the same in an animal or animal cell of interest, or in a plant or plant cell. The DNA may be nuclear DNA or organellar DNA, and is preferably organellar DNA of a plant.

The present invention may relate to (1) to (51) below based on the above-described contents, but is not limited thereto.
(1) A base editing composition capable of converting adenine (A) in DNA to guanine (G), the composition comprising one or more fusion proteins, wherein each fusion protein comprises a DNA binding protein and a cytosine deaminase, at least one of the one or more fusion proteins comprises an adenine deaminase, at least one of the one or more fusion proteins comprises uracil DNA glycosylase (UDG), and the cytosine deaminase is present in full-length form or as two splits.
(2) The base editing composition of (1), wherein the DNA is nuclear DNA or organellar DNA.
(3) The base editing composition of (1) or (2), wherein the cytosine deaminase is selected from the group consisting of APOBEC (apolipoprotein B editing complex), AID (activation-induced deaminase), DddAtox, and variants thereof.
(4) The base editing composition of any one of (1) to (3), wherein the cytosine deaminase comprises the amino acid sequence of SEQ ID NO: 2 in full-length form, and one or more amino acids selected from the group consisting of positions 37, 59, 109, and 129 of SEQ ID NO: 2 are substituted with different amino acids.
(5) The base editing composition of (4), wherein the substitution is selected from the group consisting of: S at position 37 substituted with G, G at position 59 substituted with S, A at position 109 substituted with V, and S at position 129 substituted with G.
(6) The base editing composition of any one of (1) to (3), wherein the cytosine deaminase is present as first and second splits, wherein one fusion protein comprises the first split, and another fusion protein comprises the second split, and wherein one or more amino acids located on the dimerization interface of the first and second splits are substituted with different amino acids.
(7) The base editing composition of (6), herein the first split of the cytosine deaminase comprises the amino acid sequence of SEQ ID NO: 2 from the N-terminus up to a residue selected from G at position 33, G at position 44, A at position 54, N at position 68, G at position 82, N at position 98, or G at position 108, and the second split of the cytosine deaminase comprises the amino acid sequence of SEQ ID NO: 2 from a residue selected from G at position 34, P at position 45, G at position 55, N at position 69, T at position 83, A at position 99, or A at position 109 to the C-terminus.
(8) The base editing composition of (6), wherein the first split of the cytosine deaminase comprises the amino acid sequence of SEQ ID NO: 5, the second split of the cytosine deaminase comprises the amino acid sequence of SEQ ID NO: 6, and one or more amino acids selected from the group consisting of positions 87, 88, 91, 92, 95, 100, 101, 102, and 103 of SEQ ID NO: 5, and positions 13, 14, 15, and 16 of SEQ ID NO: 6 are substituted with different amino acids.
(9) The base editing composition of any one of (1) to (8), wherein the adenine deaminase is selected from the group consisting of APOBEC, AID, TadA, and variants thereof.
(10) The base editing composition of any one of (1) to (9), wherein each fusion protein comprises a DNA binding protein selected from the group consisting of a zinc finger protein, a TALE (transcription activator-like effector) protein, and a CRISPR-associated nuclease.
(11) The base editing composition of any one of (1) to (10), wherein the DNA is organellar DNA, and each fusion protein comprises a nuclear export signal (NES).
(12) The base editing composition of any one of (1) to (11), wherein the DNA is mitochondrial DNA, and each fusion protein comprises a mitochondrial targeting sequence (MTS).
(13) The base editing composition of any one of (1) to (11), wherein the DNA is chloroplast DNA, chromoplast DNA, or leucoplast DNA, and each fusion protein comprises a chloroplast transit signal (CTS).
(14) The base editing composition of any one of (1) to (10), wherein the DNA is nuclear DNA, and each fusion protein comprises a nuclear localization signal (NLS).
(15) The base editing composition of any one of (1) to (14), wherein the composition does not substantially result in the conversion of cytosine (C) to thymine (T).
(16) The base editing composition of any one of (1) to (14), wherein the conversion of cytosine (C) to thymine (T) occurs at a frequency of less than 20%.
(17) The base editing composition of any one of (1) to (14), wherein the conversion of cytosine (C) to thymine (T) occurs at a frequency of less than 10%.
(18) The base editing composition of any one of (1) to (14), wherein the conversion of cytosine (C) to thymine (T) occurs at a frequency of less than 5%.
(19) The base editing composition of any one of (1) to (14), wherein the conversion of cytosine (C) to thymine (T) occurs at a frequency of less than 1%.
(20) A base editing composition capable of converting adenine (A) to guanine (G) in the DNA of a plant cell, the composition comprising a fusion protein comprising a DNA binding protein, a cytosine deaminase (preferably a full-length cytosine deaminase), and an adenine deaminase (wherein the full-length cytosine deaminase preferably comprises the amino acid sequence of SEQ ID NO: 9).
(21) The base editing composition of (20), wherein the DNA is nuclear DNA or organellar DNA.
(22) The base editing composition of (20) or (21), wherein the cytosine deaminase is APOBEC (apolipoprotein B editing complex), AID (activation-induced deaminase), or DddAtox, or a variant thereof.
(23) The base editing composition of any one of (20) to (22), wherein the cytosine deaminase comprises the amino acid sequence of SEQ ID NO: 2, with one or more amino acids selected from the group consisting of positions 37, 59, 109, and 129 of the amino acid sequence of SEQ ID NO: 2 substituted with different amino acids.
(24) The base editing composition of (23), wherein S at position 37 is substituted with G, G at position 59 is substituted with S, A at position 109 is substituted with V, and/or S at position 129 is substituted with G.
(25) The base editing composition of any one of (20) to (24), wherein the adenine deaminase is selected from the group consisting of APOBEC, AID, TadA, and variants thereof.
(26) The base editing composition of any one of (20) to (25), wherein the fusion protein comprises a DNA binding protein selected from the group consisting of a zinc finger protein, a TALE protein, and a CRISPR-associated nuclease.
(27) The base editing composition of any one of (20) to (26), wherein the DNA is organellar DNA, and the fusion protein comprises a nuclear export signal (NES).
(28) The base editing composition of any one of (20) to (27), wherein the DNA is mitochondrial DNA, and the fusion protein comprises a mitochondrial targeting sequence (MTS).
(29) The base editing composition of any one of (20) to (27), wherein the DNA is chloroplast DNA, chromoplast DNA, or leucoplast DNA, and the fusion protein comprises a chloroplast transit signal (CTS).
(30) The base editing composition of any one of (20) to (26), wherein the DNA is nuclear DNA and the fusion protein comprises a nuclear localization signal (NLS).
(31) The base editing composition of any one of (20) to (30) , wherein the fusion protein comprises uracil DNA glycosylase (UDG).
(32) The base editing composition of claim 28, wherein the composition does not substantially result in the conversion of cytosine (C) to thymine (T).
(33) The base editing composition of (31), wherein the conversion of cytosine (C) to thymine (T) occurs at a frequency of less than 20%.
(34) The base editing composition of (31), wherein the conversion of cytosine (C) to thymine (T) occurs at a frequency of less than 10%.
(35) The base editing composition of (31), wherein the conversion of cytosine (C) to thymine (T) occurs at a frequency of less than 5%.
(36) The base editing composition of (31), wherein the conversion of cytosine (C) to thymine (T) occurs at a frequency of less than 1%.
(37) A polynucleotide encoding any one of the fusion proteins contained in the base editing composition of any one of claims (1) to (36), or a combination of two or more of such polynucleotides.
(38) A base editing composition comprising the vector of (37).
(39) A base editing composition comprising the vector of (38).
(40) A plant cell transformed with the vector of claim 32 or comprising the polynucleotide or combination of polynucleotides of (37).
(41) A plant grown from the plant cell of (40).
(42) A progeny or clone of the plant of (41).
(43) A seed obtained from the plant of (41) or (42).
(44) The plant cell of (40), the plant of (41) or (42), or the seed of (43), wherein adenine (A) in the wild-type DNA has been converted to guanine (G).
(45) The plant cell, plant, or seed of (44), wherein cytosine (C) in the wild-type DNA has not been edited.
(46) A method of editing adenine (A) to guanine (G) in DNA of a plant organelle, comprising expressing the base editing composition of any one of (1) to (36), or the polynucleotide or combination of polynucleotides of (37), in a target plant or plant cell of interest.
(47) The method of (46), wherein the composition does not substantially result in the conversion of cytosine (C) to thymine (T).
(48) The method of (46), wherein the conversion of cytosine (C) to thymine (T) occurs at a frequency of less than 20%.
(49) The method of (46), wherein the conversion of cytosine (C) to thymine (T) occurs at a frequency of less than 10%.
(50) The method of (46), wherein the conversion of cytosine (C) to thymine (T) occurs at a frequency of less than 5%.

Hereinafter, the present invention is described in detail by the following examples. However, the following examples are only intended to illustrate the present invention, and the present invention is not limited thereto.

### Example 1: Transformation of psaA base editor in Arabidopsis thaliana

Transgenic plants were produced by cloning DNA encoding the following base editors targeting the psaA gene in the chloroplast of Arabidopsis thaliana and by transformation using Agrobacterium. The genetic sequence composition used is as follows.

**Table 1**

| **Base editor** | **Sequence composition** |
|---|---|
| Split TALED 1 (left) | CTS-3xFlag-Left TALE 1-Linker 1-1397N |
| Split TALED 1 (right) | CTS-3xFlag-Right TALE 2-Linker 1-1397C-Linker 2-ABE8.0 |
| Split TALED 2 (left) | CTS-3xFlag-Left TALE 1-Linkder 1-1397N-UDG |
| Split TALED 2 (right) | CTS-3xFlag-Right TALE 2-Linker 1-1397C-Linker 2-ABE8.0 |
| Split TALED 3 (left) | CTS-3xFlag-Left TALE 1-Linker 1-1397N |
| Split TALED 3 (right) | CTS-3xFlag-Right TALE 2-1397C-Linker 2-ABE8.0-Linker 2-UDG |
| Split TALED 4 (left) | CTS-3xFlag-Left TALE 1-1397N-UDG |
| Split TALED 4 (right) | CTS-3xFlag-Right TALE 2-1397C-Linker 2-ABE8.0-Linker 2-UDG |
| Split TALED 5 (left) | CTS-3xFlag-Left TALE 1-1397C-Linker 2-ABE8.0 |
| Split TALED 5 (right) | CTS-3xFlag-Right TALE 2-Linker 1-1397N |
| Split TALED 6 (left) | CTS-3xFlag-Left TALE 1-Linker 1-1397C-Linker 2-ABE8.0-Linker 2-UDG |
| Split TALED 6 (right) | CTS-3xFlag-Right TALE 2-Linker 1-1397N |
| Split TALED 7 (left) | CTS-3xFlag-Left TALE 1-Linker 1-1397C-Linker 2-ABE8.0 |
| Split TALED 7 (right) | CTS-3xFlag-Right TALE 2-Linker 1-1397N-UDG |
| Split TALED 8 (left) | CTS-3xFlag-Left TALE 1-Linker 1-1397C-Linker 2-ABE8.0-Linker 2-UDG |
| Split TALED 8 (right) | CTS-3xFlag-Right TALE 2-Linker 1-1397N-UDG |
| Monomer TALED 1 | CTS-3xFlag-Right TALE 2-Linker 2-ABE8.0-Linker 3-GSVG |
| Monomer TALED 2 | CTS-3xFlag-Right TALE 2-Linker 2-ABE8.0-Linker 3-GSVG-Linker 2-UDG |

The sequences of CTS, Left TALE, Right TALE, ABE8.0, GSVG, and UDG used above are as follows.
CTS (chloroplast transport signal):
   MDSQLVLSLKLNPSFTPLSPLFPFTPCSSFSPSLRFSSCYSRRLYSPVTVYAAK (SEQ ID NO: 8)
ABE8.0 (adenine deaminase): 
SEVEFSHEYWMRHALTLAKRARDEREVPVGAVLVLNNRVIGEGWNRAIGLH
DPTAHAEIMALRQGGLVMQNYRLIDATLYVTFEPCVMCAGAMIHSRIGRVVFGVRNS
KRGAAGSLMNVLNYPGMNHRVEITEGILADECAALLCDFYRMPRQVFNAQKKAQSS
IN (SEQ ID NO: 1)
1397N (DddAtox split): 
GSGSYALGPYQISAPQLPAYNGQTVGTFYYVNDAGGLESKVFSSGGPTPYPN
YANAGHVEGQSALFMRDNGISEGLVFHNNPEGTCGFCVNMTETLLPENAKMTVVPP
EG (SEQ ID NO: 5)
1397C (DddAtox split):
   GSAIPVKRGATGETKVFTGNSNSPKSPTKGGC (SEQ ID NO: 6)
GSVG (full-length DddAtox variant): 
GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC (SEQ ID NO: 9)
UDG (uracil DNA glycosylase): 
MASSTPKTLMDFFQPAKRLKASPSSSFPAVSVAGGSRDLGSVANSPPRVTVT
TSVADDSSGLTPEQIARAEFNKFVAKSKRNLAVCSERVTKAKSEGNCYVPLSELLVEES
WLKALPGEFHKPYAKSLSDFLEREIITDSKSPLIYPPQHLIFNALNTTPFDRVKTVIIGQD
PYHGPGQAMGLSFSVPEGEKLPSSLLNIFKELHKDVGCSIPRHGNLQKWAVQGVLLL
NAVLTVRSKQPNSHAKKGWEQFTDAVIQSISQQKEGVVFLLWGRYAQEKSKLIDATK
HHILTAAHPSGLSANRGFFDCRHFSRANQLLEEMGIPPIDWQL (SEQ ID NO: 7)
Left TALE 1 (comprising an N-terminal domain and a C-terminal domain): 
DLRTLGYSQQQQEKIKPKVRSTVAQHHEALVGHGFTHAHIVALSQHPAALGT
VAVKYQDMIAALPEATHEAIVGVGKQWSGARALEALLTVAGELRGPPLQLDTGQLLK
IAKRGGVTAVEAVHAWRNALTGAPLNLTPAQVVAIASHDGGKQALETVQRLLPVLCQ
AHGLTPAQVVAIASNGGGKQALETVQRLLPVLCQDH
GLTPDQVVAIASNNGGKQALETVQRLLPVLCQDHGLTPAQVVAIASNNGGKQALETV
QRLLPVLCQDHGLTPEQVVAIASNGGGKQALETVQRLLPVLCQA
HGLTPAQVVAIASNGGGKQALETVQRLLPVLCQDHGLTPAQVVAIASHDGGKQALET
VQRLLPVLCQDHGLTPDQVVAIASHDGGKQALETVQRLLPVLCQ
AHGLTPEQVVAIASNNGGKQALETVQRLLPVLCQAHGLTPAQVVAIASNNGGKQALE
TVQRLLPVLCQAHGLTPDQVVAIASHDGGKQALETVQRLLPVLC
QDHGLTPAQVVAIASNNGGKQALETVQRLLPVLCQAHGLTPDQVVAIASNIGGKQAL
ETVQRLLPVLCQAHGLTPDQVVAIASNIGGKQALETVQRLLPVL
CQAHGLTPAQVVAIASHDGGKQALETVQRLLPVLCQAHGLTPEQVVAIASNNGGKQA
 
LETVQRLLPVLCQAHGLTPDQVVAIASNIGGKQALETVQRLLPVLCQDHGLTPDQVV
AIASNIGGKQALETVQRLLPVLCQAHGLTPEQVVAIASNGGGKQALESIVAQLSRPDPA
LAALTNDHLVALACLGGRPALDAVKKGLG (SEQ ID NO: 10)
Right TALE (comprising an N-terminal domain and a C-terminal domain):

DLRTLGYSQQQQEKIKPKVRSTVAQHHEALVGHGFTHAHIVALSQHPAALGT
VAVKYQDMIAALPEATHEAIVGVGKQWSGARALEALLTVAGELRGPPLQLDTGQLLK
IAKRGGVTAVEAVHAWRNALTGAPLNLTPEQVVAIASNNGGKQALETVQRLLPVLCQ
AHGLTPDQVVAIASNGGGKQALETVQRLLPVLCQDHGLTPAQVVAIASNNGGKQALE
TVQRLLPVLCQAHGLTPEQVVAIASNGGGKQALETVQRLLPVLCQAHGLTPAQVVAI
ASNGGGKQALETVQRLLPVLCQAHGLTPDQVVAIASNNGGKQALETVQRLLPVLCQ
DHGLTPAQVVAIASNGGGKQALETVQRLLPVLCQDHGLTPAQVVAIASHDGGKQALE
TVQRLLPVLCQAHGLTPEQVVAIASHDGGKQALETVQRLLPVLCQAHGLTPAQVVAI
ASNNGGKQALETVQRLLPVLCQAHGLTPDQVVAIASNNGGKQALETVQRLLPVLCQ
DHGLTPAQVVAIASNIGGKQALETVQRLLPVLCQAHGLTPAQVVAIASNIGGKQALET
VQRLLPVLCQDHGLTPDQVVAIASNIGGKQALETVQRLLPVLCQDHGLTPDQVVAIAS
NNGGKQALETVQRLLPVLCQAHGLTPAQVVAIASNIGGKQALETVQRLLPVLCQAHG
LTPAQVVAIASNNGGKQALETVQRLLPVLCQDHGLTPAQVVAIASNNGGKQALETVQ
RLLPVLCQAHGLTPEQVVAIASNGGGKQALESIVAQLSRPDPALAALTNDHLVALACL
GGRPALDAVKKGLG (SEQ ID NO: 11)

The sequences of linkers used are as follows.
Linker 1:
   GS
Linker 2:
   SGSETPGTSESATPES (SEQ ID NO: 12)
Linker 3:
   LVGS

Specifically, in order to correct the psaA gene present in the chloroplast genome of Arabidopsis using the above genetic constructs, the constructs were first designed to be positioned between the RPS5A promoter, which induces expression during embryonic development, and the 35S terminator, so as to induce correction from the early stage of development. The constructs were then cloned, using Gibson assembly, Golden Gate, restriction enzymes, and the like, into a vector suitable for transforming Agrobacterium tumefaciens, a strain capable of delivering genetic constructs into the Arabidopsis nuclear genome via T-DNA. Using the Agrobacterium strain GV3101 transformed with such a vector, Arabidopsis thaliana Columbia-0 (Col-0) plants were transformed by the floral dipping technique according to a known method (Zhang et al., Nat. Protoc. 1, 641-646 (2006)).

### Example 2: Confirmation of base correction and measurement of correction efficiency through sequencing

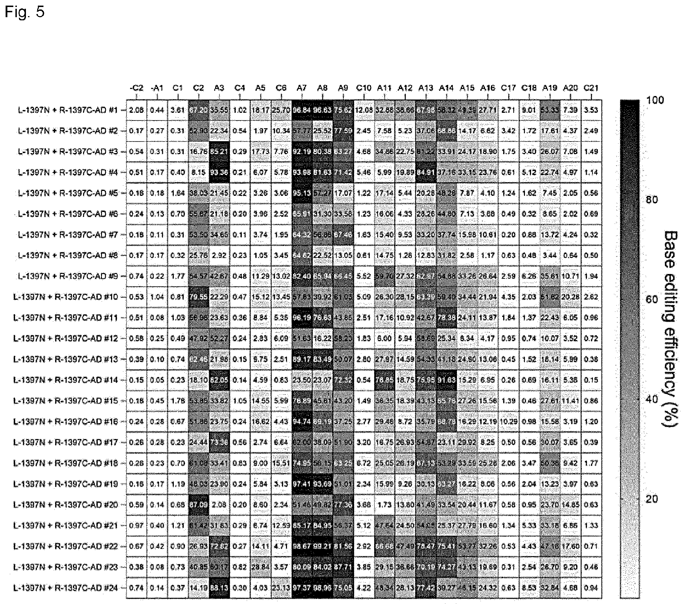

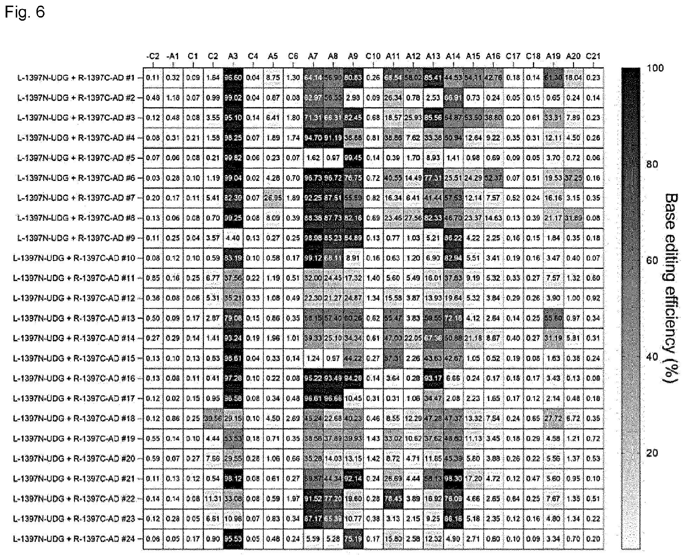

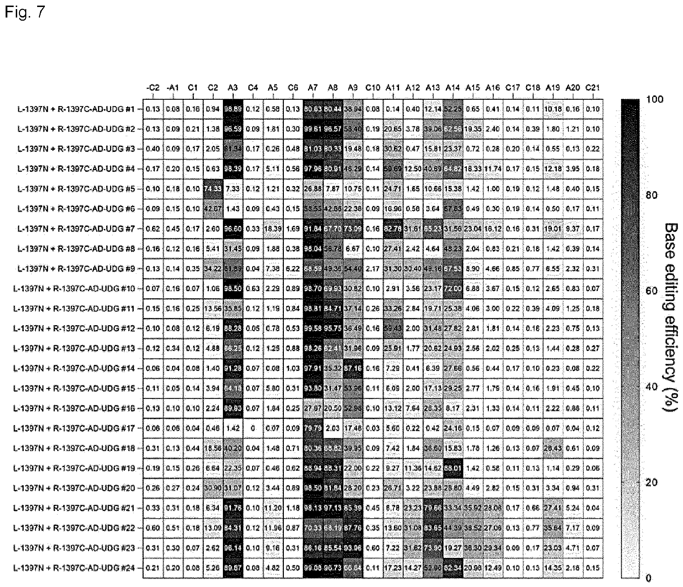

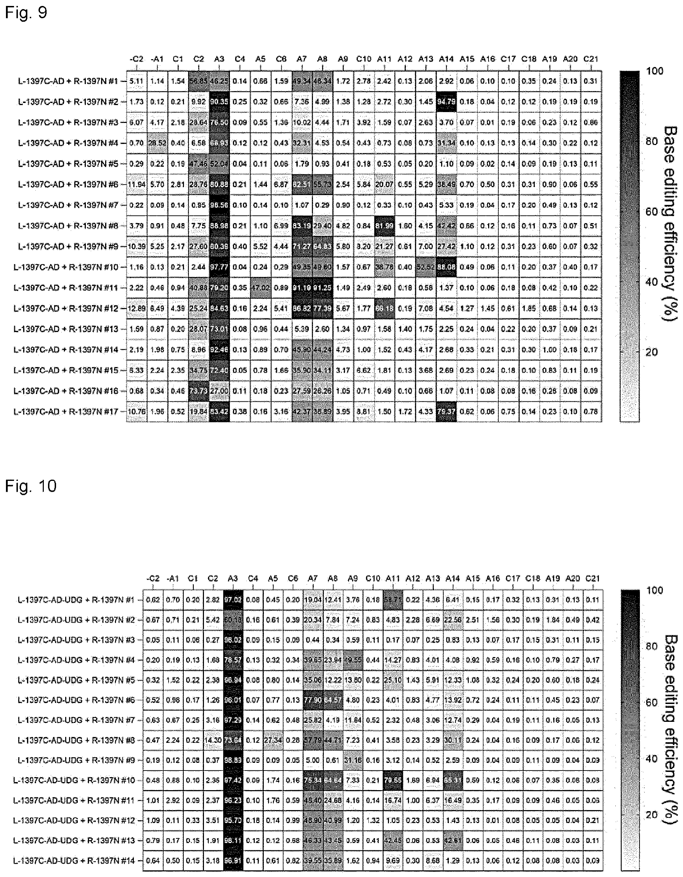

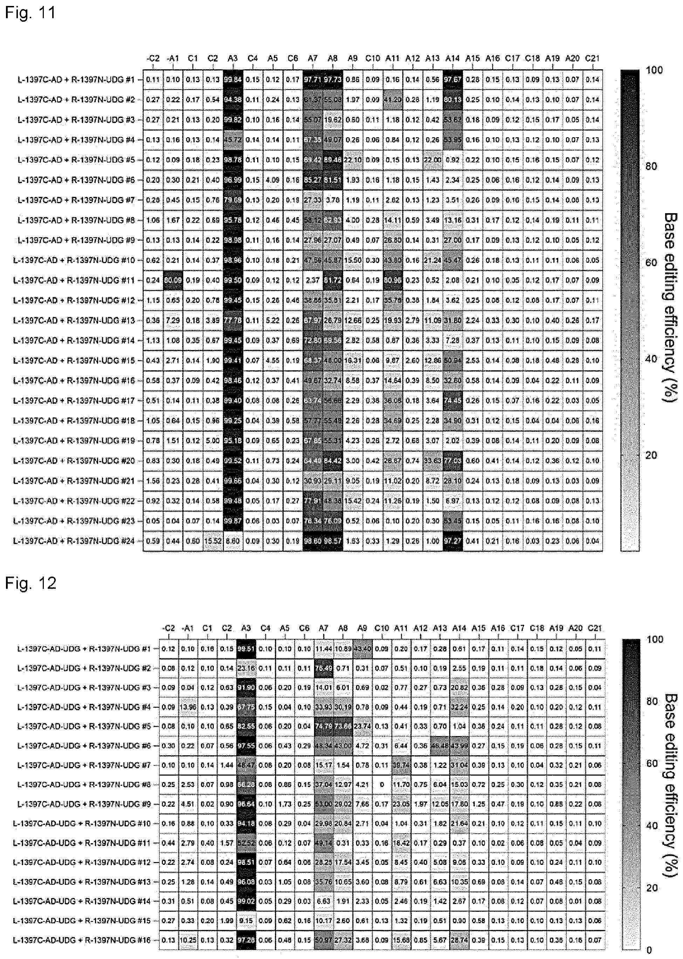

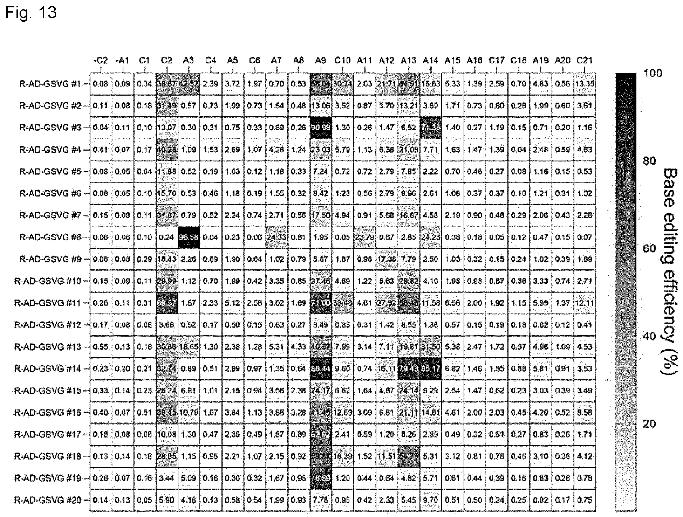

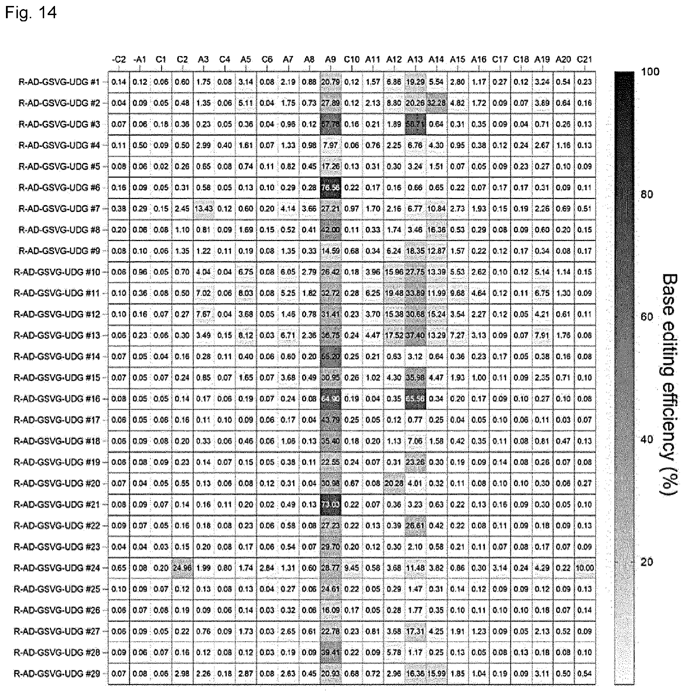

DNA was extracted from untransformed wild-type Col-0 individuals and individuals derived from first-generation seeds of transformed Arabidopsis, and the sequences were analyzed using a targeted deep sequencing method. The base editing efficiency (frequency) was calculated as the percentage of sequencing reads that reflected the results of the desired base correction among the total sequencing reads (FIG. 3 to 14).

As a result of the experiment, all the base editors according to the present invention corrected the adenine base in the target region of the psaA gene with high efficiency as shown in FIGS. 1 and 2, and in particular, it was confirmed for the first time that the adenine base of the chloroplast DNA of a plant cell was effectively corrected even when using a monomeric TALED linked to a full-length variant of DddAtox cytosine deaminase (GSVG variant) in which the toxicity was removed.

In addition, to evaluate the extent to which unwanted C-to-T corrections were avoided when using UDG-linked TALED, the average frequency of corrections at the C2 base among the correction target sites of the psaA gene was summarized in the table below for each base editor used.

**Table 2**

| **Base editor** | **C2 base editing efficiency in the psaA gene (average)** |
|---|---|
| Control (Col-0) | 0.17% |
| L-1397N + R-1397C-AD | 46.14% |
| L-1397N + R-1397C-AD-UDG | 11.72% |
| L-1397N-UDG + R-1397C-AD | 4.53% |
| L-1397N-UDG + R-1397C-AD-UDG | 1.54% |
| L-1397C-AD + R-1397N | 26.38% |
| L-1397C-AD-UDG + R-1397N | 3.21% |
| L-1397C-AD + R-1397N-UDG | 1.47% |
| L-1397C-AD-UDG + R-1397N-UDG | 0.7% |
| R-AD-GSVG | 23.96% |
| R-AD-GSVG-UDG | 1.37% |

As shown in the table above, in all of the base editors used, C-to-T correction was markedly reduced when UDG was additionally linked, and was almost equivalent to being unobserved in the wild-type Col-0 (control) in which no base editing occurred.

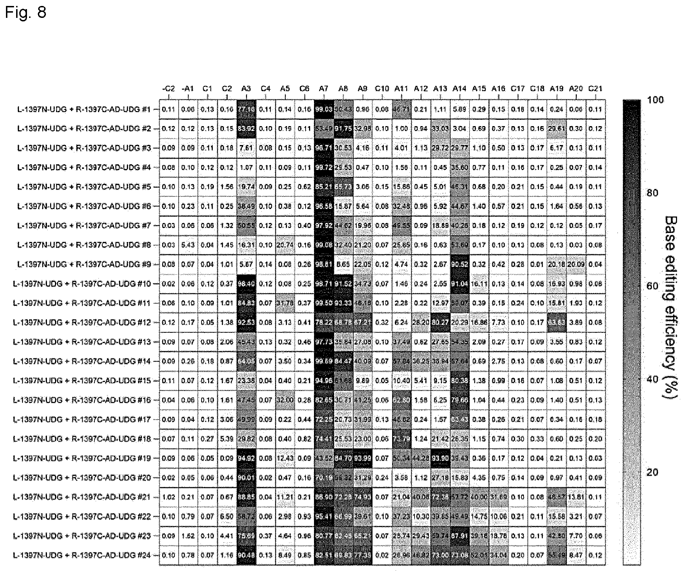

The experimental results also demonstrated that, when using the UDG-linked TALED according to the present invention, plants in which only adenine bases were corrected to guanine without correction of cytosine bases were obtained (e.g., plants #4, #10, and #14 in FIG. 8).

### Example 3: Transformation of Arabidopsis thaliana psbA base editor

In order to edit the base sequence 5'-AGT-3' encoding serine at position 264 in the psbA gene in the chloroplast of Arabidopsis thaliana to 5'-GGT-3' encoding glycine, fusion proteins were produced as shown in Table 3. Correction of the above serine at position 264 to glycine may result in resistance to the herbicide atrazine. To express the fusion protein, the following DNA was cloned and used for transformation via Agrobacterium to obtain transgenic plants.

**Table 3**

| Fusion protein | Composition |
|---|---|
| 1 | CTS-3xFlag-Left TALE 1-linker 11-TadA8e-linker 12-GSVG-linker 13-UDG |
| 2 | CTS-3xFlag-Right TALE 2-linker 11-TadA8e-linker 12-GSVG-linker 13-UDG |
| 3 | CTS-3xFlag-Left TALE 1-linker 14-1397N- linker 13UDG-RPS5A promoter-CTS-3xFlag-Right TALE 1-linker 14-1397C-linker 13-TadA8e-linker 13-UDG |
| 4 | CTS-3xFlag-Left TALE 1-linker 14-1397N- linker 13-UDG-RPS5A promoter-CTS-3xFlag-Right TALE 2-linker 14-1397C-linker 13-TadA8e-linker 13-UDG |
| 5 | CTS-3xFlag-Left TALE 2-linker 14-1397N-linker 13-UDG-RPS5A promoter-CTS-3xFlag-Right TALE 1-linker 14-1397C-linker 13-TadA8e-linker 13-UDG |
| 6 | CTS-3xFlag-Left TALE 2-linker 14-1397N-linker 13-UDG-RPS5A promoter-CTS-3xFlag-Right TALE 2-linker 14-1397C-linker 13-TadA8e-linker 13-UDG |
| 7 | CTS-3xFlag-Left TALE 1-linker 14-1397C-linker 13-TadA8e-linker 13-UDG-RPS5A promoter-CTS-3xFlag-Right TALE 1-linker 14-1397N-linker 13-UDG |
| 8 | CTS-3xFlag-Left TALE 1-linker 14-1397C-linker 13-TadA8e-linker 13-UDG-RPS5A promoter-CTS-3xFlag-Right TALE 2- linker 14-1397N-linker 13-UDG |
| 9 | CTS-3xFlag-Left TALE 2-linker 14-1397C-linker 13-TadA8e-linker 13-UDG-RPS5A promoter-CTS-3xFlag-Right TALE 2-linker 14-1397N-linker 13-UDG |

The sequences of fusion protein components used above are as follows.
CTS (chloroplast transport signal)
   MDSQLVLSLKLNPSFTPLSPLFPFTPCSSFSPSLRFSSCYSRRLYSPVTVYAAK
   (SEQ ID NO: 8)
1397N (DddAtox split) 
GSGSYALGPYQISAPQLPAYNGQTVGTFYYVNDAGGLESKVFSSGGPTPYPN
YANAGHVEGQSALFMRDNGISEGLVFHNNPEGTCGFCVNMTETLLPENAKMTVVPP
EG (SEQ ID NO: 5)
1397C (DddAtox split)
   GSAIPVKRGATGETKVFTGNSNSPKSPTKGGC (SEQ ID NO: 6)
GSVG (full-length DddAtox variant) 
GSYALGPYQISAPQLPAYNGQTVGTFYYVNDAGGLEGKVFSSGGPTPYPNYA
NAGHVESQSALFMRDNGISEGLVFHNNPEGTCGFCVNMTETLLPENAKMTVVPPEG
VIPVKRGATGETKVFTGNSNGPKSPTKGGC (SEQ ID NO: 9)
AD(TadA8e) 
SEVEFSHEYWMRHALTLAKRARDEREVPVGAVLVLNNRVIGEGWNRAIGLH
DPTAHAEIMALRQGGLVMQNYRLIDATLYVTFEPCVMCAGAMIHSRIGRVVFGVRNS
KRGAAGSLMNVLNYPGMNHRVEITEGILADECAALLCDFYRMPRQVFNAQKKAQSS
IN (SEQ ID NO: 1)
UDG (uracil DNA glycosylase) 
MASSTPKTLMDFFQPAKRLKASPSSSFPAVSVAGGSRDLGSVANSPPRVTVT
TSVADDSSGLTPEQIARAEFNKFVAKSKRNLAVCSERVTKAKSEGNCYVPLSELLVEES
WLKALPGEFHKPYAKSLSDFLEREIITDSKSPLIYPPQHLIFNALNTTPFDRVKTVIIGQD
PYHGPGQAMGLSFSVPEGEKLPSSLLNIFKELHKDVGCSIPRHGNLQKWAVQGVLLL
NAVLTVRSKQPNSHAKKGWEQFTDAVIQSISQQKEGVVFLLWGRYAQEKSKLIDATK
HHILTAAHPSGLSANRGFFDCRHFSRANQLLEEMGIPPIDWQL(SEQ ID NO: 7)
Linker 11
   GSSGSETPGTSESATPES (SEQ ID NO: 13)
Linker 12
   LVGS
Linker 13
   SGSETPGTSESATPES (SEQ ID NO: 18)
Linker 14
   GS
psbA Left 1 TALE protein
   (SEQ ID NO: 14; comprising an N-terminal domain and a C-terminal domain)
psbA Left 2 TALE protein
   (SEQ ID NO: 15; comprising an N-terminal domain and a C-terminal domain)
psbA Right 1 TALE protein
   (SEQ ID NO: 16; comprising an N-terminal domain and a C-terminal domain)
psbA Right 2 TALE protein
   (SEQ ID NO: 17; comprising an N-terminal domain and a C-terminal domain)

Specifically, in order to correct the base sequence of 5'-AGT-3' encoding serine at position 264 in the gene psbA present in the Arabidopsis thaliana chloroplast genome using the above genetic construct, it was designed to be positioned between the RPS5A promoter and the 35S terminator, which induce expression during embryonic development, so as to induce correction from the early stage of development, and then cloned using Gibson assembly, Golden Gate, restriction enzymes, and the like into a vector suitable for transforming Agrobacterium tumefaciens, a strain capable of delivering the genetic construct to the Arabidopsis nuclear genome using T-DNA, and Arabidopsis thaliana Columbia (Col-0) plants were transformed with the Agrobacterium strain GV3101 harboring such a vector by the floral dipping technique according to a known method (Zhang et al., Nat. Protoc. 1, 641-646 (2006)).

Approximately 15,000 to 20,000 seeds of Arabidopsis, each of which had the 12 types of fusion proteins introduced through the floral dipping technique, were sown in soil, and after 7 and 14 days, atrazine was applied (40 g per hectare) to secure the surviving first-generation transformed plants and analyze the base correction efficiency, and as a result, it was confirmed that A-to-G base correction was achieved when the fusion protein base editors comprising UDG were used according to the present invention (FIG. 16).

## Claims

1. A base editing composition capable of converting adenine (A) to guanine (G) in DNA, the composition comprising one or more fusion proteins,
wherein each fusion protein comprises a DNA binding protein and a cytosine deaminase,
at least one of the one or more fusion proteins comprises an adenine deaminase,
at least one of the one or more fusion proteins comprises uracil DNA glycosylase (UDG), and
the cytosine deaminase is present in full-length form or as two splits.

2. The base editing composition of claim 1, wherein the DNA is nuclear DNA or organellar DNA.

3. The base editing composition of claim 1 or 2, wherein the cytosine deaminase is selected from the group consisting of APOBEC (apolipoprotein B editing complex), AID (activation-induced deaminase), TadA (tRNA-specific adenosine deaminase), DddAtox, and variants thereof.

4. The base editing composition of any one of claims 1 to 3, wherein the cytosine deaminase comprises the amino acid sequence of SEQ ID NO: 2 in full-length form, and one or more amino acids selected from the group consisting of positions 37, 59, 109, and 129 of SEQ ID NO: 2 are substituted with different amino acids.

5. The base editing composition of claim 4, wherein the substitution is selected from the group consisting of:
S at position 37 substituted with G,
G at position 59 substituted with S,
A at position 109 substituted with V, and
S at position 129 substituted with G.

6. The base editing composition of any one of claims 1 to 3,
wherein the cytosine deaminase is present as first and second splits,
wherein one fusion protein comprises the first split, and another fusion protein comprises the second split,
and wherein one or more amino acids located on the dimerization interface of the first and second splits are substituted with different amino acids.

7. The base editing composition of claim 6, wherein the first split of the cytosine deaminase comprises the amino acid sequence of SEQ ID NO: 2 from the N-terminus up to a residue selected from G at position 33, G at position 44, A at position 54, N at position 68, G at position 82, N at position 98, or G at position 108, and
the second split of the cytosine deaminase comprises the amino acid sequence of SEQ ID NO: 2 from a residue selected from G at position 34, P at position 45, G at position 55, N at position 69, T at position 83, A at position 99, or A at position 109 to the C-terminus.

8. The base editing composition of claim 6, wherein the first split of the cytosine deaminase comprises the amino acid sequence of SEQ ID NO: 5,
the second split of the cytosine deaminase comprises the amino acid sequence of SEQ ID NO: 6, and
one or more amino acids selected from the group consisting of positions 87, 88, 91, 92, 95, 100, 101, 102, and 103 of SEQ ID NO: 5, and positions 13, 14, 15, and 16 of SEQ ID NO: 6 are substituted with different amino acids.

9. The base editing composition of any one of claims 1 to 8, wherein the adenine deaminase is selected from the group consisting of APOBEC, AID, TadA, and variants thereof.

10. The base editing composition of any one of claims 1 to 9, wherein each fusion protein comprises a DNA binding protein selected from the group consisting of a zinc finger protein, a TALE (transcription activator-like effector) protein, and a CRISPR-associated nuclease.

11. The base editing composition of any one of claims 1 to 10, wherein the DNA is organellar DNA, and each fusion protein comprises a nuclear export signal (NES).

12. The base editing composition of any one of claims 1 to 11, wherein the DNA is mitochondrial DNA, and each fusion protein comprises a mitochondrial targeting sequence (MTS).

13. The base editing composition of any one of claims 1 to 11, wherein the DNA is chloroplast DNA, chromoplast DNA, or leucoplast DNA, and each fusion protein comprises a chloroplast transit signal (CTS).

14. The base editing composition of any one of claims 1 to 10, wherein the DNA is nuclear DNA, and each fusion protein comprises a nuclear localization signal (NLS).

15. The base editing composition of any one of claims 1 to 14, wherein the composition does not substantially result in the conversion of cytosine (C) to thymine (T).

16. The base editing composition of any one of claims 1 to 14, wherein the conversion of cytosine (C) to thymine (T) occurs at a frequency of less than 20%, less than 10%, less than 5%, or less than 1%.

17. A base editing composition capable of converting adenine (A) to guanine (G) in the DNA of a plant cell, the composition comprising a fusion protein comprising a DNA binding protein, a cytosine deaminase, and an adenine deaminase.

18. The base editing composition of claim 17, wherein the DNA is nuclear DNA or organellar DNA.

19. The base editing composition of claim 17 or 18, wherein the cytosine deaminase is selected from the group consisting of APOBEC, AID, TadA, DddAtox, and variants thereof.

20. The base editing composition of any one of claims 17 to 19, wherein the cytosine deaminase comprises the amino acid sequence of SEQ ID NO: 2, with one or more amino acids selected from the group consisting of positions 37, 59, 109, and 129 of the amino acid sequence of SEQ ID NO: 2 substituted with different amino acids.

21. The base editing composition of claim 20, wherein
S at position 37 is substituted with G,
G at position 59 is substituted with S,
A at position 109 is substituted with V, and/or
S at position 129 is substituted with G.

22. The base editing composition of any one of claims 17 to 21, wherein the adenine deaminase is selected from the group consisting of APOBEC, AID, TadA, and variants thereof.

23. The base editing composition of any one of claims 17 to 22, wherein the fusion protein comprises a DNA binding protein selected from the group consisting of a zinc finger protein, a TALE protein, and a CRISPR-associated nuclease.

24. The base editing composition of any one of claims 17 to 23, wherein the DNA is organellar DNA, and the fusion protein comprises a nuclear export signal (NES).

25. The base editing composition of any one of claims 17 to 24, wherein the DNA is mitochondrial DNA, and the fusion protein comprises a mitochondrial targeting sequence (MTS).

26. The base editing composition of any one of claims 17 to 24, wherein the DNA is chloroplast DNA, chromoplast DNA, or leucoplast DNA, and the fusion protein comprises a chloroplast transit signal (CTS).

27. The base editing composition of any one of claims 17 to 23, wherein the DNA is nuclear DNA, and the fusion protein comprises a nuclear localization signal (NLS).

28. The base editing composition of any one of claims 17 to 27, wherein the fusion protein comprises uracil DNA glycosylase (UDG).

29. The base editing composition of claim 28, wherein the composition does not substantially result in the conversion of cytosine (C) to thymine (T).

30. The base editing composition of claim 28, wherein the conversion of cytosine (C) to thymine (T) occurs at a frequency of less than 20%, less than 10%, less than 5%, or less than 1%.

31. A polynucleotide encoding any one of the fusion proteins contained in the base editing composition of any one of claims 1 to 30, or a combination of two or more of such polynucleotides.

32. A vector comprising the polynucleotide or combination of polynucleotides of claim 31.

33. A base editing composition comprising the vector of claim 32.

34. A plant cell transformed with the vector of claim 32 or comprising the polynucleotide or combination of polynucleotides of claim 31.

35. A plant grown from the plant cell of claim 34.

36. A progeny or clone of the plant of claim 35.

37. A seed obtained from the plant of claim 35 or 36.

38. The plant cell of claim 34, the plant of claim 35 or 36, or the seed of claim 37, wherein adenine (A) in the wild-type DNA has been converted to guanine (G).

39. The plant cell, plant, or seed of claim 38, wherein cytosine (C) in the wild-type DNA has not been edited.

40. A method of editing adenine (A) to guanine (G) in DNA of a plant organelle, comprising expressing the base editing composition of any one of claims 1 to 30, or the polynucleotide or combination of polynucleotides of claim 31, in a target plant or plant cell of interest.

41. The method of claim 40, wherein the composition does not substantially result in the conversion of cytosine (C) to thymine (T).

42. The method of claim 40, wherein the conversion of cytosine (C) to thymine (T) occurs at a frequency of less than 20%, less than 10%, less than 5%, or less than 1%.
